# EUROPEAN PATENT SPECIFICATION

(11) **EP 1 569 634 B1**
(45) Date of publication and mention of the grant of the patent: **16.05.2007**
(21) Application number: 03780062.0
(22) Date of filing: 26.11.2003
(51) Int. Cl.: A61K 31/192, A61K 31/216, A61K 31/195, A61P 3/00, A61K 31/155

(54) **USE OF A PPAR-ALPHA AGONIST AND METFORMIN FOR THE TREATMENT OF OBESITY**
ZUSAMMENSETZUNG VON EINEM PPAR-ALPHA AGONISTEN UND METFORMIN ZUR BEHANDLUNG VON OBESITAS
UTILISATION D'UN AGONISTE PPAR-ALPHA ET METFORMINE POUR LE TRAITEMENT DE L'OBESITÉ

(30) Priority: 28.11.2002 EP 02292940
(43) Date of publication of application: 07.09.2005
(73) Proprietor: Fournier Laboratories Ireland Limited, Carrigtwohill, Co. Cork (IE)
(72) Inventor: JUNIEN, Jean-Louis, F-92310 SEVRES (FR); EDGAR, Alan, F-21490 SAINT-JULIEN (FR)
(74) Representative: Hart Davis, Jason
(86) International application number: PCT/EP2003/013302
(87) International publication number: WO 2004/047831

(56) References cited:
- WO-A-98/05331
- WO-A-99/40904
- DE-A- 2 357 875
- FR-A- 2 275 199
- FR-A- 2 796 940
- DE SILVA S.R. ET AL: "Metformin and clofibrate in maturity onset diabetes mellitus: Advantages of combined treatment." DIABETE ET METABOLISME, 5/3 (223-229). CODEN: DIMEDU, 1979, XP008014771
- CHEMICAL ABSTRACTS, vol. 133, no. 15, 9 October 2000 (2000-10-09), Columbus, Ohio, US; abstract no.: 202853, HUO, JIAXIN ET AL: "Effect of metformin on the lipid metabolism in patients with hyperlipidemia complicated with insulin resistance" XP002237098 & GUANGXI YIKE DAXUE XUEBAO, 16(6), 761-763, 1999,

## Description

The present invention is directed to the use of a PPARα agonist selected from gemfibrozil, fenofibric acid, bezafibrate, ciprofibrate and fenofibrate and metformin for the treatment of obesity.

PPARα is a subtype of the PPAR (Peroxisome Proliferator Activated Receptor) family. PPARα is predominantly expressed in tissues catabolizing high amounts of fatty acids, such as liver, heart and brown adipose tissue. Activated PPARs form heterodimers with RXR (Retinoid X Receptor) and the heterodimer binds to a specific response element, termed PPRE (PPAR Response Element), in the regulatory regions of target genes and subsequently alters their transcription. The majority of the genes whose expression is under control of PPARα code for proteins involved in intra- and extracellular lipid metabolism, such as acyl coA oxidase, acyl-coA synthetase and apolipoproteins A-I, A-II and C-III.

Fibrates can be cited as PPARα activators or agonists. In the present invention, the term agonist or activator is used equally to designate a compound that can activate a PPAR receptor.

Fibrates have been documented to lower plasma triglycerides and cholesterol levels and to be beneficial in the prevention of ischemic heart disease in individuals with dyslipidemia. They can also modestly decrease elevated fibrinogen and PAI-1 levels. Fibrate compounds, e.g., gemfibrozil, fenofibrate, bezafibrate, and ciprofibrate, elevate the level of plasma HDL cholesterol.

Metformin is mainly known for its anti-hyperglycaemic activity and is widely used in the treatment of non-insulin-dependent diabetes. In the case of insulin-dependent diabetes, metformin is also administered to the patient in combination with insulin.

EP 1054665 discloses a combination of metformin and of a fibrate chosen from fenofibrate and bezafibrate for the treatment of non-insulin-dependent diabetes. The synergistic effect observed lies in a marked improvement of hypoglycaemia.

FR 2 275 199 describes a metformin salt of clofibric acid. DE 2 357 875 also describes clofibric acid salts of metformin (or buformin). WO 98/05331 describes combination treatments involving a PPARγ agonist such as a thiazolidinedione compound and a PPARα agonist such as a fibrate.

The present invention is directed to the use of a PPARα agonist selected from gemfibrozil, fenofibric acid, bezafibrate, ciprofibrate and fenofibrate and metformin for the treatment of obesity.

The present invention is further directed to the use of a PPARα agonist selected from gemfibrozil, fenofibric acid, bezafibrate, ciprofibrate and fenofibrate, metformin and a pharmaceutically acceptable carrier for the manufacture of a pharmaceutical formulation for the treatment of obesity.

By "PPARα agonist" is meant a compound or composition which when combined with PPARα directly or indirectly (preferably binding directly to PPARα) stimulates or increases an *in vivo* or *in vitro* reaction typical for the receptor, e.g. transcriptional regulation activity, as measured by an assay known to one skilled in the art, including, the "co-transfection" or "cistrans" assays described or disclosed in U.S. Patent Nos. 4,981,784, 5,071,773, 5,298,429, 5,506,102, WO89/05355, WO91/06677, WO92/05447, WO93/11235, WO93/23431, WO94/23068, WO95/18380, CA 2,034,220, and Lehmann, et al., J. Biol. Chem. 270:12953-12956 (1995). PPARα agonists may also be identified according to an assay described in US Patent 6,008,239.

PPARα compounds are disclosed in Tontonez et al., Cell 79:1147-1156 (1994), Lehmann et al., J. Biol. Chem. 270(22):1-4, 1995, Amri et al., J. Lipid Res. 32:1449-1456 (1991), Kliewer et al., Proc. Natl. Acad. Sci. USA 94:4318-4323 (1997), Amri et al., J. Lipid Res. 32:1457-1463, (1991) and Grimaldi et al., Proc. Natl. Acad. Sci. USA 89:10930-10934 (1992). PPARα agonist compounds are also described in US Patent 6,008,239, WO97/27847, WO97/27857, WO97/28115, WO97/28137 and WO97/28149. Certain fibrate compounds are also described in WO92/10468 and WO01/80852.

Fibric acid derivatives lower the levels of triglyceride-rich lipoproteins, such as VLDL, raise HDL levels, and have variable effects on LDL levels. The effects on VLDL levels appear to result primarily from an increase in lipoprotein lipase activity, especially in muscle. This leads to enhanced hydrolysis of VLDL triglyceride content and an enhanced VLDL catabolism. Fibric acid agents also may alter the composition of the VLDL, for example, by decreasing hepatic production of apoC-III, an inhibitor of lipoprotein lipase activity. These compounds are also reported to decrease hepatic VLDL triglyceride synthesis, possibly by inhibiting fatty acid synthesis and by promoting fatty acid oxidation.

Fenofibrate is commercially available as Tricor^{™} capsules. Each capsule contains 67 mg of micronized fenofibrate.

Clofibrate is commercially available as Atromid-S capsules. Each capsule contains 500 mg of clofibrate. Clofibrate lowers elevated serum lipids by reducing the very low-density lipoprotein fraction rich in triglycerides. Serum cholesterol may be decreased. It may inhibit the hepatic release of lipoproteins (particularly VLDL) and potentiate the action of lipoprotein lipase. The recommended daily dose of clofibrate is 2 g, administered in divided doses.

Gemfibrozil is commercially available as Lopid tablets. Each tablet contains 600 mg of gemfibrozil. Gemfibrozil is a lipid regulating agent that decreases serum triglycerides and very low density lipoprotein cholesterol, and increases high density lipoprotein cholesterol. The recommended daily dose of gemfibrozil is 1200 mg, administered in two divided doses.

According to the present invention, the PPARα agonist used is either fenofibrate or a compound selected from the group consisting of one of the following or a pharmaceutically acceptable salt thereof: gemfibrozil, fenofibric acid, bezafibrate and ciprofibrate.

According to the present invention, the preferred PPARα agonist is fenofibrate, fenofibric acid or a pharmaceutically acceptable salt of fenofibric acid.

According to the invention, metformin can be administered in the form of one of its pharmaceutically acceptable salts, such as the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octanoate, decanoate, hexadecanoate, octadecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate or phosphate.

Among these salts, the hydrochloride, fumarate, embonate and chlorophenoxyacetate are more particularly preferred.

The pharmaceutically acceptable salts of metformin are obtained in a manner which is known per se by the action of metformin on the corresponding acid.

In another embodiment, the invention relates to the manufacture of a medicament for treating obesity wherein the treatment comprises coadministering an effective dosage of a PPARα agonist and metformin, where the effective dosage of the PPARα agonist is in the range of 10 to 3000 mg per day, preferably in the range of 50 to 300 mg per day.

In a further embodiment, the invention relates to the manufacture of a medicament for treating obesity wherein the treatment comprises coadministering an effective dosage of a PPARα agonist and metformin, where the effective dosage of metformin is in the range of about 10 to about 3000 mg per day, preferably in the range of about 100 to about 1000 mg per day.

According to an embodiment of the invention, the amount of metformin or of its salt which is used is from one to twenty times the mass of the PPARα agonist, preferably from one to five times and better from two to five times.

In another embodiment, the PPARα agonist and the metformin are to be administered simultaneously or co-administered.

In another embodiment, the PPARα agonist and the metformin are to be administered sequentially.

As used in this application, "co-administration" means the administration of two or more compounds to the same patient, within a time period of up to two to twelve hours. For example, co-administration encompasses (1) simultaneous administration of a first and second compound; (2) administration of a first compound, followed by administration of a second compound about 2 hours after administration of the first compound; and (3) administration of a first compound, followed by administration of a second compound about 12 hours after administration of the first compound. As described herein, the present invention encompasses co-administration of a PPARα agonist and metformin to a patient.

According to the present invention, a pharmaceutical formulation is defined as the formulation of the active compound with encapsulating material as a carrier providing a capsule in which the active component with or without other carriers, is surrounded by a carrier, which is thus in association with it. This includes tablets, powders, capsules, pills, cachets, and lozenges which can be used as solid dosage forms suitable for oral administration.

An effective dosage is defined in the present invention as the amount of a compound that prevents or ameliorates adverse conditions or symptoms of disease(s) or disorder(s) being treated. With respect to the PPARα agonist and metformin, effective dosage means a pharmacological dose in the range defined above. With respect to fibrates, the skilled artisan will understand and appreciate that the effective dosage of a given fibrate will vary with the potency of the fibrate.

Pharmaceutical formulations of the PPARα agonist and/or metformin can be prepared according to known methods. The preferred route of administering the PPARα agonist and metformin is mucosal administration, most preferably oral administration.

For preparing pharmaceutical compositions containing a PPARα agonist and/or metformin, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in admixture with the finely divided active component. In tablets, the active component is mixed with the carrier having the necessary binding properties in suitable proportions and compacted in the shape and size desired. The powders and tablets preferably contain from five or ten to seventy percent of the active compound. Suitable carriers are magnesium carbonate, magnesium stearate, talc, sugar, pectin, dextrin, starch, gelatin, tragacanth, methylcellulose, sodium carboxymethylcellulose, a low melting wax, cocoa butter.

Liquid form preparations include solutions, suspensions, and emulsions, for example, water or water propylene glycol solutions. For parenteral injection liquid preparations can be formulated in solution e.g. in aqueous polyethylene glycol solution.

Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artifidal and natural sweeteners, dispersants, thickeners, and solubilizing agents.

The pharmaceutical preparation is preferably in unit dosage form. In such form the preparation is subdivided into unit doses containing appropriate quantities of the active components. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The pharmaceutical preparation of the invention can also be a kit comprising two separate compositions, the first comprising the PPARα agonist and the second comprising metformin or a pharmaceutically acceptable salt thereof.

As shown in the examples, the Applicant unexpectedly found that a PPARα agonist and metformin combination can be used for the treatment of obesity.

The invention is further illustrated by the following examples, which are an illustration of some preferred features of the invention.

### EXAMPLE 1: Effect of PPARα agonist and metformin co-administration on body weight in Zucker rats

Studies were designed to evaluate the effects of fenofibrate, a PPARα agonist, and metformin as a combination therapy, on body weight in Zucker rats.

### METHOD

Male homozygous Zucker rats of 9 to 11 weeks of age and their lean controls were randomised into groups of 8, based on body weight and fasting plasma insulin levels.

The experimental groups were:
■ Lean rats, untreated;
■ Obese rats, treated with the vehicle p.o., twice daily
■ Obese rats, treated with Fenofibrate, 30 mg/kg, p.o., once daily
■ Obese rats, treated with Metformin, 150 mg/kg, p.o., twice daily
■ Obese rats, treated with Fenofibrate, 30 mg/kg, p.o., once daily and metformin, 150 mg/kg, p.o., twice daily.

Body weight was recorded every day during 30 days.

The results are summarized in Table 1.

**Table 1**

| | Body weight gain (g) | Body weight gain (% of change vs vehicle treated group) |
|---|---|---|
| Lean | 33.7 ± 3.0 | -52 |
| Vehicle | 70.5 ± 4.1 | |
| Fenofibrate | 52.0 ± 5.8 | -26 |
| Metformin | 67.2 ± 4.0 | -5 |
| Fenofibrate + Metformin | 44.4 ± 4.1 | -37 |

| | | |
|---|---|---|
| Values are expressed as mean ± SEM | | |

As shown in this example, the body weight gain is significantly lowered when Zucker rats are treated with fenofibrate and metformin. This diminution in the body weight gain is superior when the Zucker rats are treated with both fenofibrate and metformin than when they are treated with fenofibrate or metformin alone. Statistically significant differences between the combination of metformin and fenofibrate and the vehicle treated group are shown in the body weight gain (statistics: global covariance analysis followed by Dunnett's test, p<0.05).

Also, when comparing the body weight gain in Zucker rats treated with the combination of fenofibrate and metformin with both monotherapy of fenofibrate and metformin, the result is not a single addition of the effects of fenofibrate and metformin alone, but a synergy.

## Claims

1. Use of:
metformin;
a PPARα agonist, which is either fenofibrate or a compound selected from the group consisting of one of the following or a pharmaceutically acceptable salt thereof: gemfibrozil, fenofibric acid, bezafibrate and ciprofibrate; and
a pharmaceutically acceptable carrier,
in the manufacture of a pharmaceutical formulation for the treatment of obesity.

2. The use according to claim 1, wherein the PPARα agonist is fenofibrate, fenofibric acid or a pharmaceutically acceptable salt of fenofibric acid.

3. The use according to claim 1 or claim 2, wherein metformin is used in the form of one of its pharmaceutically acceptable salts, selected from the group consisting of the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octanoate, decanoate, hexadecanoate, octadecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate and phosphate.

4. The use according to claim 3, wherein metformin is used in the form of its hydrochloride, fumarate, embonate or chlorophenoxyacetate salt.

5. The use according to one of claims 1 to 4, wherein the PPARα agonist and metformin are to be administered simultaneously.

6. The use according to claim 5, wherein the pharmaceutical formulation is a solid form preparation containing metformin and the PPARα agonist in a unit dosage form, wherein the solid form preparation is selected from the group consisting of: powders, tablets, pills, capsules, cachets, lozenges, suppositories and dispersible granules.

7. The use according to claim 6, wherein the unit dosage form is a capsule, tablet, cachet or lozenge.

8. The use according to claim 6 or claim 7, wherein the solid form preparation consists of metformin, the PPARα agonist and a solid carrier consisting of or more substances acting as diluents, flavoring agents, binders, preservatives, tablet disintegrating agents, or encapsulating materials.

9. The use according to any of claims 6 to 8, wherein the solid form preparation comprises metformin and the PPARα agonist, in addition to magnesium carbonate, magnesium stearate, talc, sugar, pectin, dextrin, starch, gelatin, tragacanth, methylcellulose, sodium carboxymethylcellulose, a low melting wax or cocoa butter.

10. The use according to one of claims 1 to 4, wherein the PPARα agonist and metformin are to be administered sequentially.

11. The use according to any of claims 1 to 10, wherein the effective dosage of the PPARα agonist is in the range of 10 to 3000 mg per day, and the effective dosage of metformin is in the range of 10 to 3000 mg per day.

12. The use according to claim 11, wherein the effective dosage of the PPARα agonist is in the range of 50 to 300 mg per day, and the effective dosage of metformin is in the range of 100 to 1000 mg per day.

13. The use according to any of claims 1 to 12, wherein the amount of metformin or of its salt which is used is from one to twenty times the mass of the PPARα agonist.

14. The use according to claim 13, wherein the amount of metformin or of its salt which is used is from one to five times the mass of the PPARα agonist.

15. The use according to claim 14, wherein the amount of metformin or of its salt which is used is from two to five times the mass of the PPARα agonist.

16. Use of:
metformin; and
a PPARα agonist, which is either fenofibrate or a compound selected from the group consisting of one of the following or a pharmaceutically acceptable salt thereof: gemfibrozil, fenofibric acid, bezafibrate and ciprofibrate;
in the manufacture of a kit for the treatment of obesity, the kit comprising two separate compositions, the first comprising the PPARα agonist and the second comprising metformin or a pharmaceutically acceptable salt thereof.

17. The use according to claim 16, wherein the PPARα agonist is fenofibrate, fenofibric acid or a pharmaceutically acceptable salt of fenofibric acid.

18. The use according to claim 16, wherein the effective dosage of the PPARα agonist is in the range of 50 to 300 mg per day, and the effective dosage of metformin is in the range of 100 to 1000 mg per day.

## Patentansprüche

1. Verwendung von:
Metformin,
einem PPARα Agonisten, welcher entweder Fenofibrat ist oder eine Verbindung gewählt aus der Gruppe bestehend aus einem der Folgenden oder einem pharmazeutisch geeigneten Salz davon:
Gemfibrozil, Fenofibrinsäure, Bezafibrat und Ciprofibrat; und
einem pharmazeutisch geeigneten Träger,
bei der Herstellung einer pharmazeutischen Formulierung zur Behandlung von Obesitas.

2. Verwendung gemäß Anspruch 1, wobei der PPARα Agonist Fenofibrat, Fenofibrinsäure oder ein pharmazeutisch geeignetes Salz von Fenofibrinsäure ist.

3. Verwendung gemäß Anspruch 1 oder 2, wobei Metformin in Form eines seiner pharmazeutisch geeigneten Salze verwendet wird, gewählt aus der Gruppe bestehend aus Hydrochlorid, Acetat, Benzoat, Citrat, Fumarat, Embonat, Chlorphenoxyacetat, Glycolat, Palmoat, Aspartat, Methansulfonat, Maleat, Parachlorphenoxyisobutyrat, Format, Laktat, Succinat, Sulfat, Tartrat, Cyclohexancarboxylat, Hexanoat, Octanoat, Decanoat, Hexadecanoat, Octadecanoat, Benzolsulfonat, Trimethoxybenzoat, Paratoluolsulfonat, Adamantancarboxylat, Glycoxylat, Glutamat, Pyrrolidoncarboxylat, Naphthalensulfonat, 1-Glucosephosphat, Nitrat, Sulfit, Dithionat und Phosphat.

4. Verwendung gemäß Anspruch 3, wobei Metformin in Form seines Hydrochlorid-, Fumarat-, Embonat- oder Chlorphenoxyacetatsalzes verwendet wird.

5. Verwendung gemäß einem der Ansprüche 1 bis 4, wobei der PPARα Agonist und Metformin gleichzeitig verabreicht werden.

6. Verwendung gemäß Anspruch 5, wobei die pharmazeutische Formulierung eine Präparation in fester Form ist, welche Metformin und den PPARα Agonisten in Form einer Dosierungseinheit aufweist, wobei die Präparation in fester Form gewählt ist aus einer Gruppe bestehend aus: Pulvern, Tabletten, Pillen, Kapseln, Kapseln aus Stärkemasse, Pastillen, Zäpfchen und dispergierbaren Granulaten.

7. Verwendung gemäß Anspruch 6, wobei die Form einer Dosierungseinheit eine Kapsel, Tablette, eine Kapsel aus Stärkemasse oder eine Pastille ist.

8. Verwendung gemäß Anspruch 6 oder 7, wobei die Präparation in fester Form aus Metformin, dem PPARα Agonisten und einem festen Träger besteht, der aus einer oder mehreren Substanzen besteht, die als Verdünner, Aromastoffe, Bindemittel, Konservierungsmittel, Mittel zum Auflösen von Tabletten oder Einkapselungsmaterialien wirken.

9. Verwendung gemäß einem der Ansprüche 6 bis 8, wobei die Präparation in fester Form Metformin und den PPARα Agonisten zusätzlich zu Magnesiumcarbonat, Magnesiumstearat, Talk, Zucker, Pektin, Dextrin, Stärke, Gelatine, Tragant, Methylzellulose, Natriumcarboxymethylzellulose, einem niedrig schmelzenden Wachs oder Kakaobutter aufweist.

10. Verwendung gemäß einem der Ansprüche 1 bis 4, wobei der PPARα Agonist und Metformin nacheinander verabreicht werden.

11. Verwendung gemäß einem der Ansprüche 1 bis 10, wobei die wirksame Dosierung des PPARα Agonisten im Bereich von 10 bis 3000 mg pro Tag liegt und die wirksame Dosierung von Metformin im Bereich von 10 bis 3000 mg pro Tag liegt.

12. Verwendung gemäß Anspruch 11, wobei die wirksame Dosierung des PPARα Agonisten im Bereich von 50 bis 300 mg pro Tag liegt und die wirksame Dosierung von Metformin im Bereich von 100 bis 1000 mg pro Tag liegt.

13. Verwendung gemäß einem der Ansprüche 1 bis 12, wobei die Menge an Metformin oder dessen Salz, die verwendet wird, das Ein- bis Zwanzigfache des Gewichts des PPARα Agonisten beträgt.

14. Verwendung gemäß Anspruch 13, wobei die Menge an Metformin oder dessen Salz, die verwendet wird, das Ein- bis Fünffache des Gewichts des PPARα Agonisten beträgt.

15. Verwendung gemäß Anspruch 14, wobei die Menge an Metformin oder dessen Salz, die verwendet wird, das Zwei- bis Fünffache des Gewichts des PPARα Agonisten beträgt.

16. Verwendung von:
Metformin; und
einem PPARα Agonisten, welcher entweder Fenofibrat ist oder eine Verbindung gewählt aus der Gruppe bestehend aus einem der Folgenden oder einem pharmazeutisch geeigneten Salz davon:
Gemfibrozil, Fenofibrinsäure, Bezafibrat und Ciprofibrat
bei der Herstellung eines Kits zur Behandlung von Obesitas, wobei das Kit zwei getrennte Zusammensetzungen aufweist, wobei die erste den PPARα Agonisten aufweist und die zweite Metformin oder ein pharmazeutisch geeignetes Salz davon aufweist.

17. Verwendung gemäß Anspruch 16, wobei der PPARα Agonist Fenofibrat, Fenofibrinsäure oder ein pharmazeutisch geeignetes Salz von Fenofibrinsäure ist.

18. Verwendung gemäß Anspruch 16, wobei die wirksame Dosierung des PPARα Agonisten im Bereich von 50 bis 300 mg pro Tag liegt und die wirksame Dosierung von Metformin im Bereich von 100 bis 1000 mg pro Tag liegt.

## Revendications

1. Utilisation de :
la metformine ;
un agoniste du PPARα, qui est soit le fénofibrate, soit un composé choisi dans le groupe constitué par un des produits ou des sels pharmaceutiquement acceptables des produits suivants : gemfibrozil, acide fénofibrique, bézafibrate et ciprofibrate ; et
un support pharmaceutiquement acceptable;
dans la fabrication d'une formulation pharmaceutique pour le traitement de l'obésité.

2. Utilisation selon la revendication 1, dans laquelle l'agoniste de PPARα est le fénofibrate, l'acide fénofibrique ou un sel pharmaceutiquement acceptable de l'acide fénofibrique.

3. Utilisation selon la revendication 1 ou la revendication 2, dans laquelle la metformine est utilisée sous la forme d'un de ses sels pharmaceutiquement acceptables, choisis dans le groupe constitué par le chlorhydrate, l'acétate, le benzoate, le citrate, le fumarate, l'embonate, le chlorophénoxyacétate, le glycolate, le palmoate, l'aspartate, le méthanesulfonate, le maléate, le parachlorophénoxyisobutyrate, le formiate, le lactate, le succinate, le sulfate, le tartrate, le cyclohexanecarboxylate, l'hexanoate, l'octanoate, le décanoate, l'hexadécanoate, l'octadécanoate, le benzènesulfonate, le triméthoxybenzoate, le paratoluènesulfonate, l'adamantanecarboxylate, le glycoxylate, le glutamate, le pyrrolidonecarboxylate, le naphtalènesulfonate, le 1-glucosephosphate, le nitrate, le sulfite, le dithionate et le phosphate.

4. Utilisation selon la revendication 3, dans laquelle la metformine est utilisée sous la forme de son sel chlorhydrate, fumarate, embonate ou chlorophénoxyacétate.

5. Utilisation selon l'une quelconque des revendications 1 à 4, dans laquelle l'agoniste de PPARα et la metformine doivent être administrés simultanément.

6. Utilisation selon la revendication 5, dans laquelle la formulation pharmaceutique est une préparation sous forme solide contenant de la metformine et un agoniste de PPARα dans une forme d'administration unitaire, où la préparation sous forme solide est choisie dans le groupe constitué par : les poudres, les comprimés, les pilules, les gélules, les cachets, les pastilles, les suppositoires et les granulés à disperser.

7. Utilisation selon la revendication 6, dans laquelle la forme d'administration unitaire est une gélule, un comprimé, un cachet ou une pastille.

8. Utilisation selon la revendication 6 ou la revendication 7, dans laquelle la préparation sous forme solide est constituée par de la metformine, l'agoniste de PPARα et un support solide constitué par une ou plusieurs substances agissant en tant que diluants, agents aromatisants, liants, conservateurs, agents de délitement de comprimés ou matières d'encapsulation.

9. Utilisation selon l'une quelconque des revendications 6 à 8, dans laquelle la préparation sous forme solide comprend de la metformine et l'agoniste de PPARα, en plus de carbonate de magnésium, de stéarate de magnésium, de talc, de sucre, de pectine, de dextrine, d'amidon, de gélatine, de gomme adragante, de méthylcellulose, de carboxyméthylcellulose sodique, d'une cire à faible température de fusion ou de beurre de cacao.

10. Utilisation selon l'une quelconque des revendications 1 à 4, dans laquelle l'agoniste de PPARα et la metformine doivent être administrés successivement.

11. Utilisation selon l'une quelconque des revendications 1 à 10, dans laquelle la dose efficace de l'agoniste de PPARα est dans l'intervalle de 10 à 3000 mg par jour et la dose efficace de metformine est dans l'intervalle de 10 à 3000 mg par jour.

12. Utilisation selon la revendication 11, dans laquelle la dose efficace de l'agoniste de PPARα est dans l'intervalle de 50 à 300 mg par jour et la dose efficace de metformine est dans l'intervalle de 100 à 1000 mg par jour.

13. Utilisation selon l'une quelconque des revendications 1 à 12, dans laquelle la quantité de metformine ou de son sel qui est utilisée est de une à vingt fois la masse de l'agoniste de PPARα.

14. Utilisation selon la revendication 13, dans laquelle la quantité de metformine ou de son sel qui est utilisée est de une à cinq fois la masse de l'agoniste de PPARα.

15. Utilisation selon la revendication 14, dans laquelle la quantité de metformine ou de son sel qui est utilisée est de deux à cinq fois la masse de l'agoniste de PPARα.

16. Utilisation de :
la metformine ; et
un agoniste du PPARα, qui est soit le fénofibrate, soit un composé choisi dans le groupe constitué par un des produits ou des sels pharmaceutiquement acceptables des produits suivants : gemfibrozil, acide fénofibrique, bézafibrate et ciprofibrate ;
dans la fabrication d'un kit pour le traitement de l'obésité ; le kit comprenant deux compositions séparées, la première comprenant l'agoniste du PPARα et la seconde comprenant de la metformine ou un sel pharmaceutiquement acceptable de celle-ci.

17. Utilisation selon la revendication 16, dans laquelle l'agoniste de PPARα est le fénofibrate, l'acide fénofibrique ou un sel pharmaceutiquement acceptable de l'acide fénofibrique.

18. Utilisation selon la revendication 16, dans laquelle la dose efficace de l'agoniste de PPARα est dans l'intervalle de 50 à 300 mg par jour et la dose efficace de metformine est dans l'intervalle de 100 à 1000 mg par jour.
